(19) Europäisches Patentamt
European Patent Office
Office européen des brevets

(11) EP 1 053 345 B1

(12) **EUROPÄISCHE PATENTSCHRIFT**

(45) Veröffentlichungstag und Bekanntmachung des Hinweises auf die Patenterteilung:
**03.03.2004 Patentblatt 2004/10**

(21) Anmeldenummer: **99934214.0**

(22) Anmeldetag: **29.01.1999**

(51) Int Cl.[7]: **C12P 17/02**, C12N 9/10, C07D 305/14

(86) Internationale Anmeldenummer:
**PCT/EP1999/000599**

(87) Internationale Veröffentlichungsnummer:
**WO 1999/040216 (12.08.1999 Gazette 1999/32)**

(54) **VERFAHREN ZUR HERSTELLUNG VON BACCATIN**

METHOD FOR PRODUCING BACCATIN

PROCEDE DE PREPARATION DE BACCATINE

(84) Benannte Vertragsstaaten:
**AT BE CH CY DE DK ES FI FR GB GR IE IT LI LU MC NL PT SE**
Benannte Erstreckungsstaaten:
**RO SI**

(30) Priorität: **06.02.1998 DE 19804815**

(43) Veröffentlichungstag der Anmeldung:
**22.11.2000 Patentblatt 2000/47**

(73) Patentinhaber: **INDENA S.p.A.**
**20139 Milano (IT)**

(72) Erfinder:
- **Bombardelli, Ezio**
  **20141 Milano (IT)**
- **Menhard, Birgitta**
  **40789 Monheim (DE)**
- **Zenk, Meinhart Hans**
  **81243 Munich (DE)**

(74) Vertreter: **Huber, Bernhard, Dipl.-Chem. et al**
**Weickmann & Weickmann**
**Patentanwälte**
**Postfach 86 08 20**
**81635 München (DE)**

(56) Entgegenhaltungen:
- **CHEMICAL ABSTRACTS, vol. 130, Columbus, Ohio, US; abstract no. 207279, PENNINGTON, JASON J. ET AL: "Acetyl CoA: 10-deacetylbaccatin-III-10-O-acetyltransfe rase activity in leaves and cell suspension cultures of Taxus cuspidata" XP002103769 & PHYTOCHEMISTRY (1998), 49(8), 2261-2266 CODEN: PYTCAS;ISSN: 0031-9422,**
- **CHEMICAL ABSTRACTS, vol. 130, Columbus, Ohio, US; abstract no. 110419, LEE, DONGHYUN ET AL: "Selective enzymic acylation of 10-Deacetylbaccatin II" XP002103770 & TETRAHEDRON LETT. (1998), 39(49), 9039-9042 CODEN: TELEAY;ISSN: 0040-4039,**
- **CHEMICAL ABSTRACTS, vol. 124, Columbus, Ohio, US; abstract no. 7137, NANDURI, VENKATA B. ET AL: "Fermentation and isolation of C10-deacetylase for the production of 10-deacetylbaccatin III from baccatin III" XP002103771 & BIOTECHNOL. BIOENG. (1995), 48(5), 547-50 CODEN: BIBIAU;ISSN: 0006-3592,**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft ein Verfahren zur Herstellung von Baccatin bzw. von Baccatinderivaten durch selektive Acetylierung der entsprechenden 10-Desacetylverbindungen, ein isoliertes Enzym, welches diese Acetylierungsreaktion katalysiert sowie ein Verfahren zur Herstellung des Enzyms.

Taxol (Paclitaxel) ist ein vielversprechendes Mittel zur Behandlung von Krebs, welches antileukämische und tumorhemmende Aktivität aufweist (siehe z.B.: M. Suffnes et al., in "The Alkaloids, Chemistry and Pharmacology, A. Brossi, Hrsg. Academic Press; Orlando, FL, 1985, Vol. XXV, Kapitel 1). Ursprünglich war Taxol aus der Rinde bestimmter Eibenarten (Taxus taxaceae) gewonnen worden. Die Isólierung von Taxol aus Rinde ist jedoch schwierig und aufwendig und man erhält das gewünschte Taxol aus Rinde nur in sehr geringen Ausbeuten (40 bis 165 mg/kg) (siehe z. B. R.W. Miller et al., J. Org. Chem. 46 (1981) 1469-1474; V. Sénilh et al., J. Nat. Procl. 47 (1984) 131-137; N. Magri et al., J. Org. Chem 51 (1986) 797-802). Zudem führt die Verwendung von Rinde zum Absterben der Eiben, welche sehr langsam nachwachsen, sodass das Ausgangsmaterial nur in begrenztem Umfang zur Verfügung steht.

Nach der Entdeckung der Eigenschaften von Taxol, die es für eine Verwendung als chemotherapeutisches Mittel gegen Krebs empfehlen, wurden zahlreiche Anstrengungen unternommen, es durch synthetische oder semisynthetische Verfahren herzustellen. So wurde versucht, die Struktur des Taxols durch organische Synthese herzustellen (siehe z.B. W. F. Berkowitz et al., J. Org. Chem. 52 (1987) 1119-1124). Aufgrund der Komplexheit des Moleküls, gelang es bisher jedoch nicht, Taxol in praktisch verwendbaren Mengen durch vollständige organische Synthese herzustellen.

Ein weiterer Weg, der eingeschlagen wurde, um Taxol zu erhalten, ist eine partielle Synthese ausgehend von einem leicht und in großer Menge erhältlichen Vorläufer. Einer dieser Ansätze geht von 10-Desacetylbaccatin III aus, welches leicht und in großer Ausbeute von Blättern von Taxus baccata L extrahiert werden kann (G. Chauviere et al., Seances Acad. Sci., Ser. 2, 1981, 293, 501-503). Es ist dabei möglich, etwa 1 g 10-Desacetylbaccatin III pro kg Blätter zu isolieren, wobei die Blätter schnell nachwachsen. So können ohne weiteres große Mengen des Vorläufers 10-Desacetylbaccatin III erhalten werden.

Aus diesem aus biologischem Material gewonnenen Vorläufer kann dann durch partielle Synthese der gewünschte Wirkstoff Taxol hergestellt werden. Allerdings hat sich herausgestellt, dass, so ähnlich die Strukturen von 10-Desacetylbaccatin III und Taxol zu sein scheinen, diese partielle Synthese noch große Schwierigkeiten beinhaltet und zumeist nur unter Verwendung spezifischer Schutzgruppen erfolgreich durchgeführt werden kann, wobei das gewünschte Produkt Taxol lediglich in geringen Ausbeuten erhalten wird.

Denis et al., (J. Am. Chem. Soc. 110 (1988), 5917-5919) beschreiben die Umsetzung von 10-Desacetylbaccatin III zu Taxol in zwei Schritten. Im ersten Schritt wird 10-Desacetylbaccatin III chemisch in 10-Stellung acetyliert. Im zweiten Schritt erfolgt die Überführung von Baccatin in Taxol. Der erste Schritt ist jedoch nicht regiospezifisch, sodass insbesondere auch eine Acetylierung von 10-Desacetylbaccatin an Position 7 stattfindet. Es ist deshalb notwendig, die Hydroxygruppe an dieser Position durch eine Schutzgruppe der Acetylierung unzugänglich zu machen. Nur durch die Verwendung einer Schutzgruppe konnte eine ausschließliche Acetylierung in 10-Stellung erreicht werden. Die Verwendung einer Schutzgruppe beinhaltet jedoch zwei weitere Verfahrensschritte (Anbringen und Entfernen der Schutzgruppe), was zum einen aufwendig ist und zum anderen die Ausbeute des erhaltenen Produkts deutlich verringert. Ein weiterer Nachteil bei der Verwendung von Schutzgruppen besteht darin, dass insbesondere bei Verwendung des Produkts als pharmazeutischen Wirkstoff aufwendige Reinigungs- und Analyseverfahren nachgeschaltet werden müssen, um sicher zu stellen, dass sich keine mit Schutzgruppen versehenen Moleküle mehr im Produkt befinden.

Zocher et al. (Biochem. Biophys. Res. Commun., 229 (1996), 16-20) beschreiben eine Biosynthese von Taxol. In einem Zwischenschritt wurde dabei die Acetylierung von 10-Desacetylbaccatin III zu Baccatin III mit Hilfe von Pflanzenrohextrakten aus den Wurzeln von Taxus baccata durchgeführt. Es gelang jedoch nicht, Substanzen zu isolieren oder zu charakterisieren, die die Acetylierung bewirken. Nachteilig bei der Verwendung eines Rohextraktes istdie Tatsache, dass zahlreiche weitere Reaktionen, insbesondere Acetylierung an weiteren Positionen ebenfalls durch im Rohextrakt vorliegende Stoffe veranlasst oder beeinflusst werden können. Weiterhin weist ein Pflanzenrohextrakt keine definierte und reproduzierbare Zusammensetzung auf, sodass die Verwendung von Pflanzenrohextrakten zu nicht kontrollierbaren und variierenden Umsetzungen und Ausbeuten führt.

Eine Aufgabe der vorliegenden Erfindung war es deshalb, ein Verfahren bereitzustellen, mit dem Baccatin und dem Baccatin ähnliche Baccatinderivate durch selektive Acetylierung der entsprechenden 10-Desacetylverbindungen in Position 10 hergestellt werden können. Eine weitere Aufgabe bestand darin, eine isolierte Substanz bereitzustellen, die spezifisch diese Reaktion katalysiert.

Diese Aufgaben werden erfindungsgemäß gelöst durch ein Verfahren zur Herstellung von Baccatin oder Baccatinderivaten, welches dadurch gekennzeichnet ist, dass man 10-Desacetylbaccatin oder ein 10-Desacetylbaccatinderivat in Gegenwart eines isolierten Enzyms und eines Acetyldonors umsetzt, wobei als Enzym eine Acetyltransferase mit einem Molekulargewicht von 70 bis 72 kD, bestimmt durch SDS-PAGE (Natriumdodecylsulfat-Polyacrylamid-Gelelektrophorese), verwendet wird, die aus Zellkulturen von Taxus chinensis erhältlich ist. Es wurde festgestellt, dass eine regioselektive Acetylierung in Position 10 durch ein isoliertes Enzym katalysiert wird, welches aus Taxus chinensis Zellsuspensionskulturen gewonnen werden kann. Überraschenderweise wurde festgestellt, dass unter Verwendung des isolierten und gereinigten Enzyms eine hohe Regiospezifität hinsichtlich der Acetylierung in Position 10 erhalten werden kann. Diese Spezifität ist bevorzugt > 80 %, mehr bevorzugt > 90 % und am meisten bevorzugt > 95 %. Es wurde festgestellt, dass mit dem erfindungsgemäß verwendeten Enzym eine Spezifität > 99 % erhalten werden kann. Eine Spezifität von > 80 % bedeutet hierbei, dass die Acetylierung zu mehr als 80 % an Position 10 und zu weniger als 20 % an anderen Positionen der Ausgangsverbindung stattgefunden hat. Dies hat zur Folge, dass weitere Hydroxygruppen, die im Ausgangsstoff vorliegen, nicht mit einer Schutzgruppe blockiert werden müssen, da eine Acetylierung dieser weiteren Hydroxygruppen bei Verwendung des erfindungsgemäßen Enzyms nicht oder nur in sehr geringem Umfang auftritt.

Überraschenderweise wurde festgestellt, dass das erfindungsgemäß verwendete Enzym eine hohe Substratspezifität aufweist. So werden nur 10-Desacetylbaccatin bzw. 10-Desacetylbaccatinderivate umgesetzt, die an und in der Umgebung von der C10-Position eine dem 10-Desacetylbaccatin III ähnliche Konfiguration aufweisen. Insbesondere werden Baccatinderivate, bei denen der Zugang zur Position 10 durch raumfüllende Substituenten blockiert ist, wie etwa 10-Desacetyltaxol und 10-Desacetylcephalomannin nicht acetyliert. Eine Voraussetzung dafür, dass Baccatinderivate durch das erfindungsgemäße Enzym als Substrat erkannt werden, ist es deshalb, dass diese Derivate, die Taxan-Ringstruktur aufweisen, in den Positionen 7, 8, 9, 10, 11, 12 und 13 im Wesentlichen dem 10-Desacetylbaccatin III entsprechen, d.h. keine anderen Substituenten oder lediglich Substituenten, die ein geringes Volumen aufweisen, an diesen Positionen tragen. Bevorzugt ist das Verfahren für Baccatinderivate geeignet, die an den Positionen 7 bis 13 dieselben Substituenten wie 10-Desacetylbaccatin III oder wenigstens zum Teil Substituenten mit geringerem Volumen als die Substituenten von 10-Desacetylbaccatin III, insbesondere Wasserstoff, tragen. Voluminöse Substituenten an den übrigen Positionen stören die Reaktion nicht. Besonders bevorzugt wird das Verfahren zur Acetylierung von 10-Desacetylbaccatin III verwendet. Weiterhin wird das Verfahren besonders bevorzugt zur selektiven Acetylierung in Position 10 von 14-Hydroxy-10-desacetylbaccatin III verwendet.

Demgegenüber werden 10-Desacetylbaccatin III-Derivate, deren Hydroxygruppe in Position 7 durch eine voluminöse Schutzgruppe blockiert ist, wie etwa 7-TES-10-DAB-III oder 7-BOC-10-DAB-III vom erfindungsgemäßen Enzym nicht als Substrate erkannt. Eine solche Blockierung ist aber auch nicht nötig, da eine regioselektive Acetylierung in Position 10 auch bei Vorliegen weiterer Hydroxygruppen an anderen Positionen stattfindet.

Es ist mit dem erfindungsgemäßen Verfahren möglich, Taxanderivate selektiv in Position 10 zu acetylieren, die die gleichen, weniger oder weniger voluminöse Substituenten an den Positionen 7 bis 13 als 10-Desacetylbaccatin III aufweisen. Solche Taxanderivate, bei denen die in 10-Desacetylbaccatin III vorliegenden Substituenten (d.h. OH in Position 7, $CH_3$ in Position 8, =O in Position 9, OH in Position 10, $CH_3$ in Position 12 und OH in Position 13) vorliegen oder durch einen ähnlich voluminösen oder kleineren Rest, insbesondere Wasserstoff, ersetzt sind, fallen hierin unter den Begriff 10-Desacetylbaccatinderivate und können ebenfalls regiospezifisch acetyliert werden, soweit sie in Position 10 eine OH-Gruppe aufweisen. Beispiele solcher Derivate sind 10-Desacetyltaxuyunnanin-C, 10,14-Desacetyltaxuyunannin-C, 2,10,14-Desacetyltaxuyunannin-C, 5,10,14-Desacetyltaxuyunannin-C und 2,5,10,14-Desacetyltaxuyunannin-C.

Bevorzugt stellt man mit dem erfindungsgemäßen Verfahren Baccatin-III aus 10-Desacetylbaccatin-III, 14-Hydrobaccatin-III aus 14-Hydroxy-10-desacetylbaccatin-III oder Taxuyunnanin C aus 10-Desacetyltaxuyunnanin C her.

Das erfindungsgemäße Verfahren wird in Gegenwart eines Acetyldonors durchgeführt. Als Acetyldonor ist grundsätzlich jede Substanz geeignet, die bei der katalytischen Umsetzung der 10-Desacetylausgangssubstanz eine Acetylgruppe zur Verfügung stellt. Bevorzugt wird die Umsetzung in Gegenwart von Acetyl-Coenzym-A als Acetyldonor durchgeführt.

Die erfindungsgemäße Verwendung eines isolierten Enzyms bietet verfahrenstechnisch viele Vorteile. Die Umsetzung ist insbesondere hinsichtlich der Umsetzungsgeschwindigkeit und hinsichtlich der Reproduzierbarkeit bei der Verwendung eines isolierten Enzyms leicht zu kontrollieren.

Bevorzugt weist das verwendete Enzym einen isoelektrischen Punkt von pH 5,4 bis 5,8, bevorzugt 5,5 bis 5,7 und insbesondere von pH 5,6 auf. Weiterhin wurde festgestellt, dass das erfindungsgemäß verwendete Enzym eine Michaelis Menten Konstante $K_M$ für Acetyl-Coenzym A von 55 bis 65 µM, bevorzugt von 59 bis 63 µM und insbesondere von 61 µM aufweist.

Ein weiterer Gegenstand der Erfindung ist ein isoliertes Enzym, welches dadurch gekennzeichnet ist, dass es a) 10-Desacetylbaccatin-III in Gegenwart eines Acetyldonors, insbesondere Acetyl-Coenzym A selektiv an Position 10 acetyliert, b) ein Molekulargewicht von 70 bis 72 kD aufweist, bestimmt durch SDS-PAGE und c) aus Zellkulturen von Taxus chinensis erhältlich ist.

Das erfindungsgemäße Enzym liegt bevorzugt in einer Reinheit > 50 %, insbesondere > 80 %, mehr bevorzugt > 90 % und am meisten bevorzugt > 95 % vor. Das erfindungsgemäße Enzym zeichnet sich dadurch aus, dass es 10-Desacetylbaccatin-III in Gegenwart eines Acetyldonors, inbesondere Acetyl CoA selektiv an Position 10 acetyliert.

Dies bedeutet insbesondere, dass eine Acetylierung der übrigen Hydroxygruppen von 10-Desacetylbaccatin III an Position 1, 7 und 13 praktisch nicht beobachtet wird. Die Acetylierungsreaktion weist inbesondere eine Selektivität von > 50 % hinsichtlich Position 10, bevorzugt > 80 %, mehr bevorzugt > 90 % und am meisten bevorzugt > 95 % auf.

Das isolierte Enzym ist weiterhin durch ein Molekulargewicht von 70 bis 72 kD, bestimmt durch SDS-PAGE, charakterisiert. Zur Bestimmung des Molekulargewichts wurden 0,3 µg homogenes Protein in einem 10 %-igen denaturierenden SDS-Gel parallel mit Markerproteinen mit bekanntem Molekulargewicht (Rainbow-Marker) chromatographiert. Durch Silberfärbung wurden die Proteine sichtbar gemacht, wobei das Molekulargewicht durch Vergleich der Rf-Werte der Eichproteine und des erfindungsgemäßen Enzyms bestimmt wurde. Das durch SDS-Gelelektrophorese bestimmte Molekulargewicht wurde durch Gelfiltration an einer geeigneten Gelfiltrationssäule bestätigt. Die Gelfiltration wurde an einer mit 50 mM Tris, pH 8,5, 20 mM 2-Mercaptoethanol äquilibrierten Biosilect-SEC 250-5-Säule (Biorad) in einer FPLC-Anlage (BioLogic Workstation, Biorad) durchgeführt, und zwar bei einer Flussrate von 0,2 ml/min. Dabei wurde die Säule zunächst mit Proteinen mit bekanntem Molekulargewicht geeicht. Unter identischen Bedingungen wurden dann 50 µg des erfindungsgemäßen Proteins durch die Säule geleitet. Das Eluat wurde in Fraktionen von 250 µl gesammelt und die Aktivität des Eluats wurde, wie unten beschrieben, bestimmt. Das Molekulargewicht wurde durch Vergleich der Elutionszeiten mit den bekannten Standards ermittelt.

Das erfindungsgemäße Enzym kann aus Zellkulturen von Taxus chinensis isoliert werden. Es weist einen isoelektrischen Punkt von pH 5,4 bis 5,8, bevorzugt pH 5,5 bis 5,7 und insbesondere von pH 5,6 auf. Weiterhin wurde eine Michaelis Menten-Konstante $K_M$ für das verwendete Enzym für Acetyl-Coenzym A von 55 bis 65 µM, bevorzugt von 59 bis 63 µM und insbesondere von 61 µM gefunden.

Bei dem erfindungsgemäßen Enzym handelt es sich um eine Acetyltransferase, insbesondere um eine Acetyl-CoA-10-Hydroxytaxan-O-Acetyltransferase.

Ein weiterer Gegenstand der Erfindung ist ein Verfahren zur Herstellung des oben beschriebenen Enzyms, welches dadurch gekennzeichnet ist, dass man das Enzym aus einer das Enzym enthaltenden Quelle isoliert, indem man bekannte Reinigungsverfahren verwendet, und nach jeder Reinigung die Fraktionen bestimmt, in denen das Enzym vorliegt, indem man 10-Desacetylbaccatin oder ein 10-Desacetylbaccatinderivat und einen Acetyldonor zugibt und das gebildete Acetylierungsprodukt nachweist.

Als Enzym enthaltende Quelle kann beispielsweise ein Pflanzenextrakt verwendet werden. Bevorzugt wird als Enzym enthaltende Quelle eine Zellkultur, insbesondere eine Suspensionszellkultur verwendet. Die Verwendung einer Zellkultur ist deshalb vorteilhaft, da große Mengen an Ausgangsmaterial gewonnen werden können. Im Vergleich zur Verwendung von Rohextrakt als Enzym enthaltener Quelle ist bei Verwendung einer Zellkultur aufgrund der großen Menge an Ausgangsmaterial eine Aufreinigung des Enzyms bis zu hoher Reinheit möglich. Besonders bevorzugt wird ein Ausgangsmaterial verwendet, das von Taxus chinensis stammt, beispielsweise eine Zellkultur von Taxus chinensis.

Zur Reinigung des Enzyms aus dem Ausgangsmaterial können zur Gewinnung von Enzymen bzw. Proteinen bekannte Reinigungsverfahren verwendet werden. Bevorzugt verwendete Verfahren umfassen die Ammoniumsulfatfällung aus dem Rohextrakt sowie chromatographische Reinigungsverfahren, wie etwa die Verwendung einer Sephadex G-25 Säule, eine Anionenaustausch-Chromatographie z.B. an DEAE-Sephacel, eine Gelfiltration z.B. an Ultrogel AcA 44, eine Anionenaustausch-Chromatographie z.B. an HighQ, eine Chromatographie über eine Hydroxyapatitsäule, eine Farbstoffaffinitätschromatographie z. B. an High Trap Blue, eine hydrophobe Interaktionschromatographie z. B. an Phenylsepharose und/oder eine Farbstoffaffinitätschromatographie z.B. an Mimetic-Grün 1 A6XL. Bevorzugt umfasst die Reinigung wenigstens einen Schritt unter Verwendung einer Anionenaustausch-Chromatographie an HighQ. Die HighQ-Säule ist ein Anionenaustauscher mit $-N^{+}(CH_3)_3$-Gruppen als Liganden. Es wurde festgestellt, dass insbesondere bei diesem Reinigungsschritt Substanzen entfernt werden, die eine Acetylierung an anderen Positionen als Position 10 katalysieren.

Beim erfindungsgemäßen Verfahren wird nach jedem Reinigungsschritt die Enzymaktivität der Fraktionen bestimmt, um festzustellen, in welchen Fraktionen das Enzym vorliegt. Dazu wird die Fraktion bzw. ein Teil der Fraktion mit 10-Desacetylbaccatin oder einem 10-Dssacetylbaccatin-Derivat, wie oben definiert, und einen Acetyldonor versetzt. In den Fraktionen, in denen das gewünschte Enzym vorliegt, kann in Position 10 acetyliertes Produkt nachgewiesen werden. Für diesen Nachweis setzt man bevorzugt 10-Desacetylbaccatin-III oder 10-Desacetyltaxuyunannin-C ein. Der Nachweis des gebildeten Acetylierungsproduktes kann durch die Verwendung geeigneter Markierungsgruppen in den Ausgangssubstanzen erreicht werden. Bevorzugt wird ein markierter Acetyldonor verwendet. Ein solcher markierter Acetyldonor umfasst eine markierte Acetylgruppe, mit deren Hilfe dann in Position 10 acetyliertes Produkt bestimmt werden kann. Bevorzugt wird ein radioaktiv markierter Acetyldonor verwendet. Geeignete radioaktive Markierungsgruppen sind dabei $^{13}C$ und $^{14}C$. Besonders bevorzugt wird als Acetyldonor ein Acetyl-Coenzym A verwendet, insbesondere [2-$^{14}$C]-Acetyl-Coenzym-A.

Es ist auch möglich, den Nachweis über eine Markierung mit einem schweren Isotop durchzuführen. In diesem Fall kann das Umsetzungsprodukt mittels Massenspektrometrie bestimmt werden.

Mit dem erfindungsgemäßen Verfahren zur Herstellung von Baccatin oder Baccatinderivaten unter Verwendung des erfindungsgemäßen Enzyms ist es möglich, spezifisch in 10-Stellung acetylierte Baccatin- bzw. Taxanverbindungen herzustellen. Solche Verbindungen sind insbesondere als Ausgangssubstanzen für die partielle Synthese von Taxol von Interesse. Ein weiterer Gegenstand der Erfindung ist deshalb ein Verfahren zur Herstellung von Taxol oder/und Taxolderivaten, welches dadurch gekennzeichnet ist, dass man Baccatin- oder Baccatinderivate, welche nach dem oben beschriebenen Verfahren hergestellt wurden, nach bekannten Verfahren zu Taxol bzw. Taxolderivaten umsetzt. Die partielle Umsetzung von Baccatin bzw. Baccatinderivaten zu Taxol bzw. Taxolderivaten ist im Stand der Technik beschrieben und umfasst im Wesentlichen das Anbringen geeigneter Substituenten an der Hydroxygruppe in Position 13 der Baccatinderivate. Die hierfür geeigneterweise verwendeten Baccatinderivate weisen deshalb zumindest in Position 13 eine freie OH-Gruppe auf.

Die Umsetzung von Baccatinderivaten zu Taxol bzw. Taxolderivaten erfolgt insbesondere durch eine Veresterung der OH-Gruppe in Position 13 der Baccatinderivate mit einer geeigneten Säure. Solche Verfahren sind in der Literatur ausführlich beschrieben, beispielsweise im US-Patent 4,814,470 (Colin et al.), im US-Patent Re. 34,277 (Denis et al.), in EP 0 400 971 A2, im US-Patent 4,924,011 (Denis et al.), im US-Patent Nr. 5,476,954 (Bourzat et al.) sowie von Denis et al., J. Am. Chem. Soc. 110 (1988), 5917-5919.

Zur Veranschaulichung sind im folgenden die Strukturformeln von Baccatin-III und Paclitaxel angegeben:

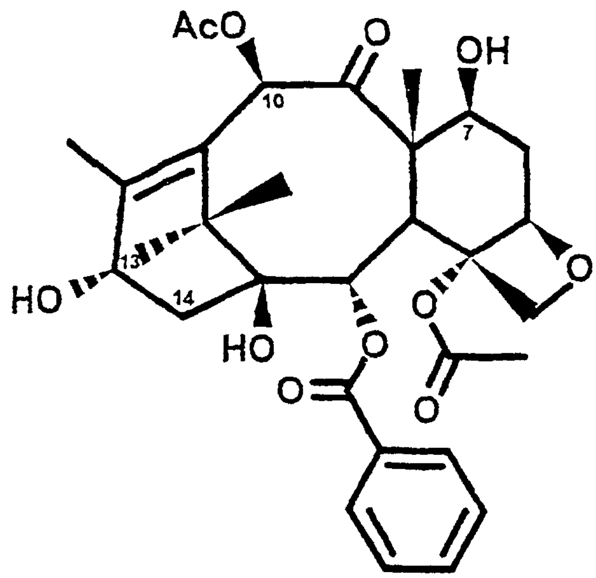

Baccatin III

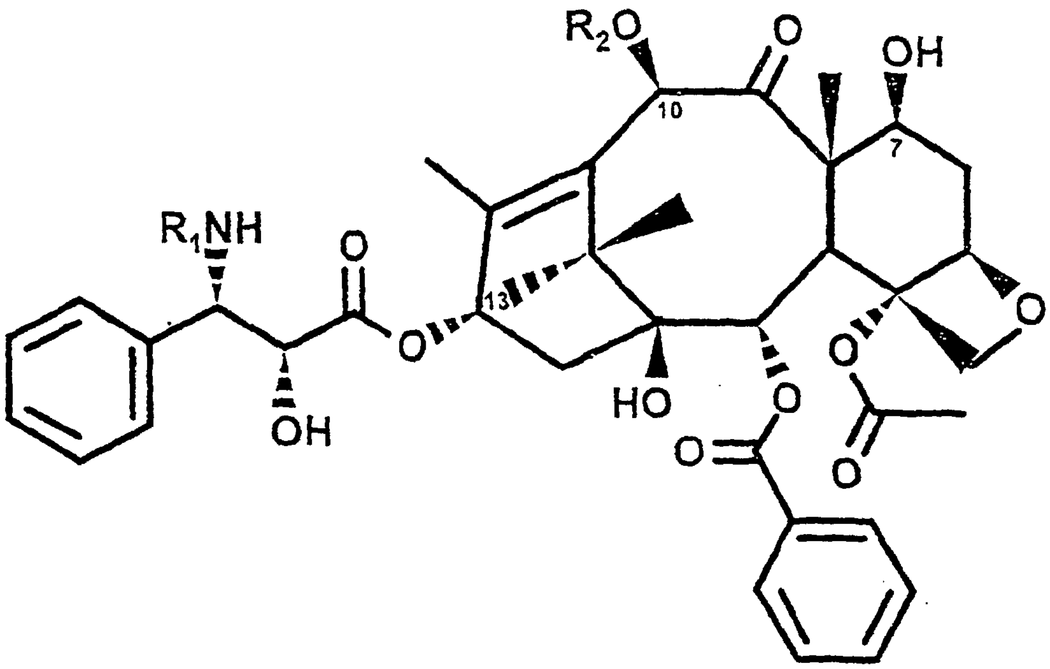

**Paclitaxel $R_1$= $COC_6H_5$ ; $R_2$= $CH_3CO$**

Die Erfindung wird durch die folgenden Beispiele weiter erläutert.

<u>Beispiel 1</u>

Kultivierung von Taxus chinensis Zellsuspensionen

Es wurden Taxus chinensis-Suspensionskulturen aus der Sammlung des Instituts für Pharmazeutische Biologie der Universität München verwendet, die ursprünglich von Nadeln eines T. chinensis Baumes stammen. Man ließ die Kulturen bei 24 °C, 100 U/min und 1500 lux 14 Tage wachsen. Dann übertrug man 150 ml Zellsuspension mit einer sterilen 50 ml-Pipette in 250 ml B5 + 1-Medium:

Zusammensetzung des B5 + 1-Mediums (modifiziert nach Gamborg, Miller, Ojima: Experimental Research, 1968, 50, pp. 151-158)

| | |
|---|---|
| Napthylessigsäure | 10 µM |
| Benzylaminopurin | 0,2 µM |

| | mg/l |
|---|---|
| $NaH_2PO_4 \cdot H_2O$ | 150 |
| $CaCl_2 \cdot 2H_2O$ | 150 |
| $(NH_4)_2SO_4$ | 134 |
| $MgSO_4 \cdot 7H_2O$ | 250 |
| $KNO_3$ | 2500 |
| $FeSO_4 \cdot 7H_2O$ | 25,6 |
| $Na_2EDTA \cdot 2H_2O$ | 34,27 |
| KJ | 0,75 |
| $MnSO_4 \cdot H_2O$ | 10 |
| $H_3BO_3$ | 3 |

(fortgesetzt)

| | mg/l |
|---|---|
| $ZnSO_4 \cdot 7H_2O$ | 3 |
| $Na_2MoO_4 \cdot 2H_2O$ | 0,25 |
| $CuSO_4 \cdot 5H_2O$ | 0,25 |
| $CoCl_2 \cdot 6H_2O$ | 0,25 |
| Nicotinsäure | 1 |
| Thiaminiumdichlorid | 10 |
| Pyridoxolhydrochlorid | 1 |
| meso-Inosit | 1000 |
| D(+)Saccharose | 20000 |
| pH 5,6 | |
| NZ-Amine | 1000 |

Die NZ-Amine wurden nach Autoklavieren und Erkalten unter sterilen Bedingungen als sterile Stocklösung (10 g/l) zum Medium gegeben.

Am 3. Tag nach diesem Überimpfen fügte man 30 μM Methyljasmonat (Fa. Serva) hinzu und ließ die Kultur weitere 4 Tage wachsen (Gundlach, H., Müller, M.J., Kutchan, T.M., Zenk, M.H., 1992, Proc. Natl. Acad. Sci. USA, 89, S. 2389-2393). Dann wurden die Zellen durch Vakuumfiltration vom Medium getrennt und nach Schockgefrieren mit flüssigem Stickstoff für den Enzymaufschluss verwendet. Bei -20 °C konnten sie aber auch bis zu einem Monat gelagert werden, danach sank die Aktivität des gesuchten Enzyms stark ab.

Beispiel 2

Enzymtest zur Bestimmung der Acetyltransferase-Aktivität

Um ein Enzym zu reinigen, charakterisieren und nachweisen zu können, ist die Verfügbarkeit von präzisen und ausreichend empfindlichen Testmethoden unerlässliche Voraussetzung. Hier war dies die Erfassung des gebildeten Produktes, z.B. des Taxuyunnanin C aus 10-Desacetyltaxuyunnanin, wofür ein einfach durchzuführendes und zuverlässiges Verfahren entwickelt wurde.

Eine ausreichende Menge der zu testenden Enzymlösung wurde zu 50 μl Trispuffer (0,8 M, pH 8,5) in ein Eppendorf Cap pipettiert. Dann fügte man 30 μl gereinigtes AcetylCoA (5 nmol nicht markiertes und 0,02 μCi-[2-$^{14}$C] Acetyl-Coenzym-A) und 15 nmol einer in DMSO gelösten 3 mM Stocklösung von 10-Desacetyltaxuyunnanin C hinzu, in der Kontrolle anstelle dieses Taxans nur die entsprechende Menge DMSO. Nach 30-minütiger Inkubation bei 35 °C wurde der Ansatz mit 20 μl 12 % $H_2SO_4$ angesäuert, das gebildete Taxuyunnanin C mit 600 μl tert.-Butylmethylether ausgeschüttelt (10 min Überkopfschüttler) und der Ansatz dann zentrifugiert (4 min 14.000 U/min, Eppendorf-Zentrifuge). Das noch vorhandene, nicht umgesetzte [2-$^{14}$C] Acetyl-CoA verblieb in der wässrigen Phase. 500 μl der organischen Phase wurden durch Abblasen mit Luft zur Trockne eingeengt, wobei gleichzeitig eventuell vorhandene [2-$^{14}$C] Essigsäure, die durch das vorhergehende Ansäuern als Säure vorlag und somit flüchtig war, entfernt werden konnte. Mittels Szintillationszähler {Multiple Purpose Szintillation Counter LS 6500, Fa. Beckmann) oder Dünnschichtradioscanner (Automatic TLC Linear Analyzer, Fa. Berthold) wurde die Art und Menge des gebildeten Produktes bestimmt. Ersteren setzte man ein, um mit den gezählten cpm-Werten den Umsatz, und damit die Aktivität des Enzyms zu ermitteln. Mit Hilfe von Dünnschichtchromatographie (DC) (LM: Chloroform:Acetonitril 7:3) und anschließender Auswertung am Radioscanner konnte überprüft werden, ob die mit dem Szintillationszähler gemessene Radioaktivität auch wirklich einem Peak in Höhe des Rf-Wertes des erwarteten Produktes entsprach.

Beispiel 3

Reinigung der Acetyltransferase

3.1. Gewebeaufschluss und Entsalzen über Sephadex G25

Zur Gewinnung des Proteinrohextraktes wurden 7 Tage alte Suspensionskulturen verwendet, die am 3. Tag

nach dem Überimpfen mit 30 µM MeJA elizitiert worden waren. Es wurden 200 g frisch abgesaugte Zellen mit flüssigem Stickstoff schockgefroren. In einem eisgekühlten Mörser wurden diese dann mit 20 g PVPP gemischt und mit 400 ml Standardpuffer A (100 mM Borsäure/NaOH, pH 8,5, 20 % Glycerin, 20 mM 2-Mercaptoethanol) unter Rühren aufgetaut. Das PVPP bindet einen Teil der im weiteren Reinigungsgang störenden Phenole, u.a. die im Extrakt enthaltenen Gerbstoffe. Der homogene Zellbrei wurde dann durch 4-lagigen Mull filtriert, und der Presssaft bei 15.000 x g zentrifugiert (10 min, SS34-Rotor).

Der eisgekühlte Überstand wurde mit 50 ml, in Standardpuffer A (100 mM Borsäure/NaOH, pH 8,5, 20 % Glycerin, 20 mM 2-Mercaptoethanol) suspendiertem Calciumphosphatgel gemischt. Die Volumenangabe gibt die Menge an Gel an, die man nach 10-minütiger Zentrifugation bei 2500 U/min erhält und entspricht 1,9 g TG $Ca_3(PO_4)_2$. 10 Minuten ließ man diese Mischung im Eisbad unter gelegentlichem Rühren stehen. Während dieser Zeit sollten Begleitstoffe an das Gel adsorbieren, wie beispielsweise die in großen Mengen enthaltenen Gerbstoffe. Die Acetyltransferase blieb in Lösung und konnte durch anschließende Zentrifugation (6000 x g, 5 min, GSA-Rotor) vom Gelmaterial getrennt werden.

Anschließend wurde das als Pellet vorliegende Gel nochmals in 75 ml Standardpuffer A aufgenommen, 5 Minuten mit einem Glasstab gerührt und nochmals 5 Minuten bei 6000 U/min (GSA-Rotor) zentrifugiert, da ein Teil der Acetyltransferase an das Gel adsorbierte und durch diese Nachbehandlung in den Überstand gelangte. Ohne Verwendung des Calciumphosphatgels ergaben sich im weiteren Reinigungsgang große Schwierigkeiten, da die dann noch enthaltenen Begleitstoffe sehr schnell die Membran der Rührzelle verstopften und die anschließend verwendeten Säulen dunkelbraun färbten und deren Bindungskapazitäten rasch verringerten.

Der vereinigte eisgekühlte Überstand wurde unter langsamen Rühren portionsweise bis zur 70 %-igen Sättigung mit Ammoniumsulfat versetzt und nach vollständiger Zugabe noch weitere 30 min langsam gerührt. Anschließend konnte das ausgefallene Protein bei 15000 U/min (10 min, SS34-Rotor) pelletiert werden. Der Niederschlag wurde in 30 ml Standardpuffer B (50 mM Tris/HCl, pH 8,5, 20 % Glycerin, 20 mM 2-Mercaptoethanol) vorsichtig resuspendiert und die Lösung unter Verwendung dieses Puffers mit Hilfe einer Sephadex G25-Säule (Pharmacia; 2,7 cm ∅ x 7 cm) entsalzt, wobei gleichzeitig 60 % des Fremdproteins abgetrennt wurden. [G25-Eluat: 82 ml, 78 mg Protein]

### 3.2 Anionenaustausch-Chromatographie an DEAE-Sephacel

Bei DEAE-Sephacel handelt es sich um eine modifizierte Cellulose, an die positiv geladene Diethylaminoethylreste gebunden sind. Ist der isoelektrische Punkt gelöster Proteine saurer als der verwendete Puffer, liegen sie als Anionen vor und können somit an das positiv geladene Säulenmaterial binden. Der Zusatz von stärkeren Anionen, wie beispielsweise $Cl^-$ oder $SO_4^{2-}$, schwächt die elektrostatische Wechselwirkung von adsorbiertem Protein und DEAE-Gruppen. Demzufolge werden die Proteinanionen gegen die anorganischen Anionen ausgetauscht und das Enzym somit eluiert.

In diesem Reinigungsschritt wurden 24,4 % des Fremdproteins abgetrennt sowie insbesondere Begleitstoffe, u.a. phenolhaltige Gerbstoffe. Das Säulenmaterial färbte sich schon beim ersten Gebrauch stark braun, konnte aber mit 1 M NaOH weitgehend gereinigt werden. Die Verschmutzung der später im Reinigungsgang eingesetzten Säulen konnte durch diesen Schritt erheblich reduziert werden.

Den Durchlauf der DEAE-Säule (2,5 cm ∅ x 5 cm) einschließlich 30 ml Standardpuffer B (50 mM Tris/HCl, pH 8,5, 20 % Glycerin, 20 mM 2-Mercaptoethnaol), mit dem die Säule nachgewaschen wurde, engte man durch Druckfiltration in einer Rührzelle (Amicon, 400 ml, Membran PM10) auf 5 ml ein.
[DEAE-Eluat: 110 ml, 59 mg Protein]

### 3.3 Gelfiltration an Ultrogel AcA 44

Das Prinzip der Gelfiltration beruht darauf, dass sich Makromoleküle, wie beispielsweise Proteine, abhängig von ihrer Größe und Form zwischen einer Matrix mit definierter Porengröße (Ausschlussvolumen) und dem sie umgebenden Flüssigkeitsraum verteilen. Moleküle, die zu groß sind, um in die Poren des Gels eindringen zu können, wandern an den Partikeln vorbei und werden dadurch schneller eluiert als mittelgroße Proteine, die zunächst von den Poren retardiert werden ohne aber in diese einzudringen. Noch kleinere Moleküle, u.a. Salzionen, gelangen zunächst in die Poren, die sie erst nach einer bestimmten Verweilzeit verlassen, weshalb sie am längsten auf der Säule bleiben.

Diese Technik ist sehr schonend, da praktisch keine Wechselwirkungen zwischen Material und Protein auftreten und keine speziellen Puffer verwendet werden müssen.

Zudem erfolgt eine vollständige Entsalzung des Eluats, da die kleineren Ionen, in diesem Fall Sulfationen, viel länger auf der Säule verweilen als das aktive Protein.

Es wurde ein Polyacrylamid-Agarose-Gel verwendet, für das ein Fraktionierbereich von 10-130 kD angegeben ist (Ultrogel AcA 44, Fa. Serva).

Die eingeengte Proteinlösung wurde an einer mit Standardpuffer B äquilibrierten Säule (2,8 cm ∅ x 100 cm)

mit einer Flussrate von 20 ml/h über Nacht aufgetrennt. Die 60 Fraktionen (a 6,5 ml) wurden auf Proteingehalt und Acetyltransferase-Aktivität getestet. Die Fraktionen mit der Hauptaktivität wurden vereinigt und weiter aufgereinigt.

Mit diesem Eluat konnte die spezifische Aktivität der Acetyltransferase gemessen werden. Das Dünnschichtchromatogramm zeigte neben deutlichen Peaks bei niedrigem Rf-Wert auch einen Peak in Höhe des Rf-Wertes von Taxuyunnanin C. [AcA-Eluat: 53 ml, 25,7 mg, die Gesamtaktivität von 724 pkat wurde als 100 % angesetzt). Durch diese Gelfiltration wurden 57 % des Fremdproteins abgetrennt.

**3.4 Anionenaustausch-Chromatographie an HighQ**

Wie die DEAE-Säule ist auch die HighQ-Säule, die $-N^+(CH_3)_3$-Gruppen als Liganden besitzt, ein Anionenaustauscher, im Gegensatz zu ersterem jedoch ein starker, d.h. über einen weiten pH-Bereich ändert sich dessen Ionisierungszustand nicht. Bei schwachen Austauschern schwankt bei verschiedenen pH-Werten der Grad der Dissoziation und somit die Austauschkapazität erheblich. Da das HighQ-Säulenmaterial aus der dicht gepackten, ca. 10 µm kleinen Harzpartikeln besteht und sich somit ein hoher Gegendruck ergibt, muss sie an einer FPLC-Anlage (Fa. Biorad) betrieben werden.

Das salzfreie AcA-Eluat (53 ml) wurde mit einer Flussgeschwindigkeit von 2 ml/min auf die mit Standardpuffer B (50 mM Tris/HCl, pH 8,5, 20 % Glycerin, 20 mM 2-Mercaptoethanol) äquilibrierte HighQ-5 ml Fertigsäule gepumpt und diese auch mit diesem Puffer nachgewaschen. Im farblosen Durchlauf befand sich bereits ein Teil des inaktiven Proteins, mit 0,07 M KCl in Standardpuffer B (50 mM Tris/HCl, pH 8,5, 20 % Glycerin, 20 mM 2-Mercaptoethanol) wurde weiteres Fremdprotein und gelbe Begleitstoffe entfernt. Bei der nächsten Stufe des Gradienten, 0,14 M KCl in Standardpuffer B (50 mM Tris/HCl, pH 8,5, 20 % Glycerin, 20 mM 2-Mercaptoethanol), wurde das aktive Protein eluiert, daneben auch gelbe Begleitstoffe. Auch das mit 1 M KCl in Standardpuffer B (50 mM Tris/HCl, pH 8,5, 20 % Glycerin, 20 mM 2-Mercaptoethanol) gewonnen Eluat war gelb gefärbt und enthielt 1/3 des aufgetragenen Proteins, jedoch ohne Acetyltransferase-Aktivität. Dieser letzte Schritt regenerierte die Säule gleichzeitig.

Ein großer Vorteil dieses Reinigungsschrittes bestand darin, dass das große Volumen des AcA-Eluates schnell und schonend auf 4 ml (4 Fraktionen a 1 ml) konzentriert werden konnte.

| Gradient | Zeit (min) | 1 M KCl in Standardpuffer B (%) |
|---|---|---|
| | 0 | 0 |
| | 35 | 0 |
| | 35 | 7 |
| | 45 | 7 |
| | 45 | 14 |
| | 55 | 14 |
| | 55 | 100 |
| | 70 | 100 |

[HighQ-Eluat: 4 ml, 8,8 mg Protein, 796 pkat]

Mit diesem Reinigungsschritt konnte ein Anreicherungsfaktor von 121 % gegenüber dem AcA-Eluat erzielt werden, bei einer 66 %-igen Fremdproteinabtrennung.

**3.5 Hydroxyapatit**

Es wurde eine CHT II-Säule (Fa. Biorad) verwendet, die mit spherischen Hydroxyapatitpartikeln, $[Ca_5(PO_4)_3OH]_2$, gefüllt war. Negativ geladene Proteine können in Anwesenheit eines niedermolaren Phosphatpuffers zunächst an die $Ca^{2+}$-Kationen binden und dann von höheren Phosphatkonzentrationen im Elutionspuffer verdrängt werden.

Basische Proteine mit hohem pl besitzen eine höhere Affinität zum Säulenmaterial als solche mit niedrigerem pl. Die Hydroxyapatitstruktur mit $Ca^{2+}$-Ionen in den positiv geladenen Zentren und $PO_4^{3-}$ in den negativ geladenen, ergibt eine gemischte Ionenaustauschtrennung. Das HighQ-Eluat wurde mit einer Geschwindigkeit von 0,5 ml/min auf die mit Phosphatpuffer (10 mM $Na_2HPO_4/NaH_2PO_4$, pH 6,8, 20 % Glycerin, 20 mM 2-Mercaptethanol) äquilibrierte CHT II-Säule (5 ml) aufgepumpt und mit 12 ml dieses Puffers nachgewaschen. Die Elution des aktiven Proteins erfolgte mit 160 mM Phosphatpuffer (20 % Glycerin, 20 mM 2-Mercaptoethanol). Auch bei weiterer Erhöhung der Phosphatkonzentration auf 400 mM konnte kein Protein mehr eluiert werden.

| Gradient | Zeit (min) | 160 mM Phosphatpuffer (%) |
|---|---|---|
| | 0 | 0 |
| | 40 | 0 |
| | 40 | 100 |
| | 70 | 100 |

Die aufgefangenen Fraktionen zu 0,5 ml wurden auf Protein und Acetyltransferaseaktivität überprüft.
[CHT II-Eluat: 1,5 ml, 0,73 mg Protein, 578 pkat]
Mit diesem Reinigungsschritt konnte ein Anreicherungsfaktor von 8,7 gegenüber dem HighQ-Eluat erzielt werden, wobei bei einer Abtrennung von Fremdprotein von 92 % ein Aktivitätsverlust von 27 % zu verzeichnen war.

### 3.6 Farbstoffaffinitätschromatographie an High Trap Blue

HiTrapBlue 1 ml (Fa. Pharmacia) enthält den synthetischen polycyclischen Farbstoff Cibacron Blue F3G-A, der an eine Agarosematrix gekoppelt wurde. Diese Liganden zeigen eine gewisse strukturelle Ähnlichkeit zu natürlich vorkommenden Molekülen, wie den Cofaktoren $NAD^+$ und $NADP^+$, was sie befähigt, Proteine stark und spezifisch zu binden, u.a. Enzyme, die Adenylat enthaltende Substanzen benötigen. Das Säulenmaterial wird deshalb auch als "gruppenspezifisch" bezeichnet. Die Spezifität wird allerdings dadurch relativiert, dass von den bisher katalogisierten Enzymen ungefähr ein Drittel ein Coenzym benötigt, das eine Nukleotidkomponente enthält. Zudem können Proteine auch unspezifisch durch elektrostatische und/oder hydrophobe Wechselwirkungen an den aromatischen Liganden binden.
Die Elution erfolgt spezifisch mit dem entsprechenden Cofaktor oder unspezifisch mit Salzlösungen.
Die Acetyltransferase, die das Phosphoadenosyldiphosphat enthaltende Acetyl-CoA bindet, adsorbierte an das blaue Säulenmaterial und wurde unspezifisch mit einem linearen KCI-Gradienten eluiert. Dazu wurde das CHT II-Eluat auf die mit Standardpuffer B äquilibrierte High Trap Blue-Säule mit einer FPLC-Anlage (Fa. Biorad) bei einer Flussgeschwindigkeit von 0,5 ml/min aufgetragen und mit diesem Puffer nachgewaschen. Die Elution des an die Säule gebundenen Proteins erfolgte bei 0,5 ml/min mit einem linearen Salzgradienten von 0-1 M KCl in Standardpuffer 8 (50 mM Tris/HCl, pH 8,5, 20 % Glycerin, 20 mM 2-Mercaptoethanol) über 30 min.

| Gradient | Zeit (min) | 1 M KCl in Standardpuffer B (%) |
|---|---|---|
| | 0 | 0 |
| | 30 | 0 |
| | 60 | 100 |

Die Fraktionsgröße betrug 0,5 ml. Aktivität enthaltende Fraktionen wurden zur weiteren Reinigung vereinigt.
[High Trap Blue-Eluat: 1,5 ml, 0,05 mg Protein, 168 pkat]
Gegenüber der CHT II-Säule konnte bei diesem Reinigungsschritt ein Anreicherungsfaktor von 4,2 erzielt werden. Bei einer Abtrennung von 93 % Fremdprotein war ein Gesamtaktivitätsverlust von 71 % zu verzeichnen.

### 3.7 Hydrophobe Interaktionschromatographie an Phenylsepharose

Gibt man zu im wässrigen Milieu gelösten Proteinen bestimmte Neutralsalze, wie beispielsweise $(NH_4)_2SO_4$ oder KCl, erhöht sich die Ionenstärke der Lösung. Unter diesen Bedingungen assoziieren die hydrophoben Bereiche auf der Oberfläche der Proteine miteinander. Genauso adsorbieren sie auch an Säulenmaterialien mit hydrophoben Liganden, es kommt demzufolge zu hydrophoben Wechselwirkungen (HIC = Hydrophobic Interaction Chromatography). Diese können anschließend wieder geschwächt werden, indem ein Elutionspuffer mit niedriger Salzkonzentration verwendet wird.
Dieses Reinigungsprinzip erforderte es demnach, das High Trap Blue Eluat auf eine Konzentration von 0,5 M Ammoniumsulfat einzustellen. Dies erfolgte durch sehr langsame portionsweise Zugabe der entsprechenden Menge eisgekühlter 1 M Ammoniumsulfat-Lösung in Standardpuffer B (50 mM Tris/HCl, pH 8,5, 20 % Glycerin, 20 mM 2-Mercaptoethanol). Anschließend trägt man die Proteinlösung auf die mit 0,5 M $(NH_4)_2SO_4$ in Standardpuffer B äquilibrierten Minisäule (1 cm ∅ x 1,3 cm) auf. Nach deren Eindringen ins Gelbett (Phenylsepharose, Fa. Pharmacia) wäscht man mit 7 ml 0,5 M $(NH_4)_2SO_4$ in Standardpuffer B (50 mM Tris/HCl, pH 8,5, 20 % Glycerin, 20 mM 2-Mercaptoethanol) nach. Zur Elution des gebundenen Proteins wird 0,1 M $(NH_4)_2SO_4$ in Standardpuffer B (50 mM Tris, HCl, pH 8,5, 20 % Glycerin, 20 mM 2-Mercaptoethanol) eingesetzt. Das Eluat wird in Fraktionen von je 0,5 ml gesammelt und die

relative Proteinkonzentration und Enzymaktivität bestimmt. Die aktivsten Fraktionen wurden vereinigt und auf Aktivität und Proteingehalt getestet.
[Phenylsepharose-Eluat: 2,5 ml, 0,001 mg, 6,3 pkat]
Als Anreicherungsfaktor gegenüber dem HiTrapBlue-Eluat wurde ein Wert von 1,9 errechnet bei einem gleichzeitigen Aktivitätsverlust von 96 % und einer Abtrennung von 98 % des Fremdproteins.

**3.8 Farbstoffaffinitätschromatographie an Mimetic-Grün 1 A-6 XL**

Wie bereits bei der High Trap Blue Säule ausgeführt, treten auch bei diesem Material Wechselwirkungen zwischen der Cofaktorbindungsstelle eines Enzyms mit dem Farbstoff-Liganden auf. Nur die cofaktorabhängigen, gebundenen Enzyme können dann mit dem Cofaktor, im vorliegenden Fall Acetyl-Coenzym-A eluiert werden. Die unspezifisch adsorbierten Proteine bleiben dabei noch auf der Säule.

Bevor das Phenylsepharose-Eluat auf die Mimetic-Grün-Säule (1 cm ∅ x 1,2 cm, Affinity Chromatography Ltd., Freeport, Ballasalla, Isle of Man) pipettiert werden konnte, mußte es zunächst entsalzt werden, was wiederum mit einer PD10-Säule (Fa. Pharmacia) geschah. Hierzu trug man die 2,5 ml Phenylsepharose-Eluat auf die PD10-Säule auf und ließ sie eindringen. Die Elution erfolgte mit Standardpuffer B (50 mM Tris/HCl, pH 8,5, 20 % Glycerin, 20 mM 2-Mercaptoethanol), wobei die ersten 0,5 ml Eluat verworfen und die weiteren 2,5 ml, die das aktive Protein enthielten, gesammelt wurden.

Diese Enzymlösung wurde auf eine mit Standardpuffer B (50 mM Tris/HCl, pH 8,5, 20 % Glycerin, 20 mM 2-Mercaptoethanol) eingewaschene Mimetic-Grün-Säule pipettiert ( 1 cm ∅ x 1,2 cm). Nachdem sie ins Gelbett eingedrungen war, wurde ohne Pumpe sukzessive mit folgenden Lösungen eluiert: 3 ml Standardpuffer B (50 mM Tris/HCl, pH 8,5, 20 % Glycerin, 20 mM 2-Mercaptoethanol), 3 ml 0,5 mM Acetyl-Coenzym-A in Standardpuffer B, 2 ml Standardpuffer B, 3 ml 1 M KCl in Standardpuffer B. Dabei wurden 1 ml-Fraktionen aufgefangen und auf Protein und Acetyl-Coenzym-A-Aktivität überprüft.

Das Acetyl-Coenzym-A-Eluat enthielt als einzige Fraktion Acetyltransferase-Aktivität. Weder im Durchlauf noch im KCl-Eluat konnte Aktivität gemessen werden.

Vor Bestimmung der Aktivität im Acetyl-CoA-Eluats musste erst der Cofaktor aus der Lösung entfernt werden. Durch den großen Pool an nicht markiertem Acetyl-Coenzym-A wäre der geringe Anteil an $^{14}C$ markiertem Acetyl-CoA so verdünnt worden, dass praktisch kein radioaktives Substrat umgesetzt worden wäre.

Eine fast vollständige Entfernung des Acetyl-Coenzym-A gelang mittels einer PD10-Säule, wie oben beschrieben. Mit dem so gewonnenen Eluat konnte der übliche Aktivitätstest durchgeführt werden. Da im PD10-Eluat jedoch noch Spuren von Acetyl-CoA aus dem Elutionspuffer vorhanden waren, also eine vollständige Entfernung nicht erreicht werden konnte, wurde im Aktivitätstest der Pool an $^{14}C$-markiertem Acetyl-CoA verdünnt und somit für die Aktivität und den Reinigungsfaktor ein zu geringer Wert erhalten.
[Grün-Eluat: 2,5 ml, 0,0001 mg Protein, 0,7 pkat]
Durch diesen Reinigungsschritt wurde das restliche Fremdprotein abgetrennt und bei 81 % Aktivitätsverlust ein Reinigungsfaktor von 1,1 erreicht.

**3.9 Zusammenfassende Darstellung der Reinigung und Dokumentation der Homogenität**

Mit dem beschriebenen Verfahren gewann man die gesuchte Acetyltransferase aus dem Rohextrakt durch 70 %-ige Ammoniumsulfatfällung und 8 säulenchromatographische Schritte. Dadurch erhielt man eine Acetyltransferase-Präparation, deren spezifische Aktivität den 280-fachen Wert des AcA-Eluates erreichte, wobei die Gesamtausbeute 0,1 % betrug. Der große Aktivitätsverlust während der Reinigung, obwohl das AcA-Eluat an einem Tag aufgearbeitet wurde, lassen sich mit der großen Instabilität des Enzyms, sowie seiner Empfindlichkeit gegenüber pH-Werten unter pH 8 und gegenüber Salzionen, v.a. Ammoniumsulfat, erklären.

Um die Reinheit der Enzympräparation zu überprüfen, wurde die denaturierende Disk-Elektrophorese in Gegenwart von SDS eingesetzt.
Nach Auftrennung des konzentrierten Eluates der Mimetic-Grün-Säule mit SDS-PAGE und anschließender Silberfärbung zeigte sich nur 1 Bande.

| Reinigungsschritt | Volumen (ml) | Gesamt-aktivität (pkat) | Gesamtprotein (mg) | Spezifische Aktivität (pkat/mg) | Ausbeute (%) | Reinigung (x-fach) |
|---|---|---|---|---|---|---|
| Rohextrakt | 278 | - | 372 | - | - | - |
| $Ca_3(PO_4)_2$ | 285 | - | 194 | - | - | - |
| SephadexG25-Eluat | 82 | - | 78 | - | - | - |
| DEAE-Eluat | 110 | - | 59 | - | - | - |
| AcA44-Eluat | 53 | 724 | 25,7 | 28 | 100 | 1 |
| HighQ-Eluat | 4 | 796 | 8,8 | 90,5 | 121 | 3,2 |
| CHT II-Eluat | 1,5 | 578 | 0,73 | 792 | 80 | 43 |
| HighTrap Blue-Eluat | 1,5 | 168 | 0,05 | 3360 | 23 | 120 |
| Phenylsepharose-Eluat | 2,5 | 6,3 | 0,001 | 6300 | 0,87 | 225 |
| Mimetic Grün-Eluat | 2,5 | 0,7 | 0,0001 | 7000 | 0,1 | 280 |

Beispiel 4

**Charakterisierung der Acetyltransferase aus Taxus chinensis Suspensionskulturen**

### 4.1 pH-Optimum

Der pH-Wert übt auf die enzymatische Aktivität großen Einfluss aus, da sich je nach Azidität der Enzymlösung die Ladungen der funktionellen Gruppen bestimmter Aminosäuren verändern. Dies hat Auswirkungen auf die Konformation des aktiven Zentrums des Enzyms und somit auf dessen Aktivität. Genauso ist das Protonierungsmuster bestimmter Substrate vom pH-Wert abhängig und kann damit gleichfalls die Enzymaktivität beeinflussen.

Zur Ermittlung des optimalen pH-Bereiches wurde die Übertragung von Acetyl-Coenzym-A auf 10-Desacetyltaxuyunnanin C bei unterschiedlichen pH-Werten, von pH 5 bis pH 11 gemessen. Hierzu wurden 5,6 pkat (1,7 µg, 50 µl) einer 120-fach angereicherten Acetyltransferase (HighTrapBlue-Eluat) in folgenden Ansätzen eingesetzt:

| | |
|---|---|
| Ansätze | 50 µl 0,8 M Tris, pH 8,5 |
| | 50 µl Acetyltransferase (5,6 pkat, 1,7 µg, 120-fach angereichert) |
| | 30 µl Acetyl-Coenzym-A (5 nmol, einschließlich 0,02 µCi[2-$^{14}$C]Acetyl-Coenzym-A |
| | 5 µl 3 mM 10-Desacetyltaxuyunnanin C (15 nmol) |
| | 50 µl Millipore Wasser |
| Inkubation | 25 min bei 35 °C. |

Die Ansätze wurden dabei ohne Acetyl-Coenzym-A 5 min vorinkubiert, danach wurde die Reaktion durch Cofaktorzugabe gestartet. Nach einer Inkubationszeit von 25 min bei 35 °C stoppte man die Reaktion durch Ansäuern und Ausschütteln mit tert.-Butylmethylether. Die im Etherextrakt enthaltene Radioaktivität wurde mittels Szintillationszähler ausgewertet.

Die Acetyltransferase-Aktivität erstreckt sich über den relativ engen Bereich von pH 8,5-9, wobei das pH-Optimum bei pH 9 liegt. Die halbmaximalen Umsatzraten sind bei pH 6,8 und pH 10,8 zu finden.

Die Reinigung wurde bei pH 8,5 durchgeführt, da sich dann bei den Affinitätssäulen weniger aktives Protein im Durchlauf befand als bei pH 9.

### 4.2 Temperaturoptimum

Neben dem deutlichen Einfluss, den der pH-Wert auf die Enzymaktivität ausübt, zeigt die Enzymaktivität auch eine starke Abhängigkeit von der Inkubationstemperatur. Zunächst nimmt mit steigender Temperatur die enzymatische Aktivität zu, ab einer bestimmten, für jedes Enzym spezifischen Temperatur, fällt sie jedoch rasch ab. Bei diesen hohen Temperaturwerten erfolgt eine Denaturierung und Inaktivierung des Enzyms. Um das Temperaturoptimum der Acetyltransferase zu bestimmen wurden 1,7 µg (5,6 pkat) des 120-fach gereinigten Enzyms bei Temperaturen von 0 °C bis 50 °C zunächst 10 min ohne Acetyl-Coenzym-A inkubiert, dann nach Cofaktorzugabe weitere 25 min. Das Abstoppen der Reaktion erfolgte durch Zugabe von 20 µl 12 % $H_2SO_4$. Danach wurde das gebildete Taxuyunnanin C mit 600 µl Ether ausgeschüttelt. Die Auswertung der gebildeten Produktmenge wurde mittels Szintillationszähler durchgeführt.

| | |
|---|---|
| Ansätze | 50 µl 0,8 M Tris, pH 8,5 |
| | 50 µl Acetyltransferase (5,6 pkat, 1,7 µg Protein, 120-fach angereichert) |
| | 30 µl Acetyl-Coenzym-A (5 nmol, einschließlich 0,02 µCi[2-$^{14}$C]AcCoA) |
| | 5 µl 3 mM 10-Desacetyltaxuyunnanin C (15 nmol) |
| | 50 µl Millipore |
| Inkubation | 25 min bei der jeweiligen Temperatur |

Das Temperaturoptimum der Acetyltransferase liegt bei 35 °C.

### 4.3 Isoelektrischer Punkt

Der isoelektrische Punkt wurde durch Chromatofokussierung ermittelt. Hierzu verwendet man einen speziellen Anionenaustauscher, z.B. Mono P HR 5/20 (Fa. Pharmacia), auf den das gewünschte Enzym bei einem pH-Wert, bei dem es als Anion vorliegt, aufgetragen wird. Unter diesen Startbedingungen bindet das Enzym an das Säulenmaterial und kann dann mit einer ampholythaltigen Puffermischung (Polybuffer 94, Fa. Pharmacia), die einen pH-Gradienten über der Säule ausbildet, eluiert werden. Das Enzym löst sich genau dann vom Anionenaustauscher, wenn der pH-Wert soweit abgesenkt ist, dass es vom anionischen in den formal ungeladenen Zustand übergeht. Dieser pH-Wert entspricht dem isoelektrischen Punkt (IEP).

Für die Acetyltransferase wurde dieser mit einer BioLogic FPLC Workstation (Fa. Biorad) an einer Mono P HR 5/20 Säule (0,5 cm ∅ x 20 cm, Fa. Pharmacia) bei einer Flussrate von 0,7 ml/min bestimmt. Diese Säule wurde mit 25 mM Imidazol/HCl pH 7,4 äquilibriert und mit HighQ-Eluat beladen. Hierzu wurden 1 ml HighQ-Eluat auf 100 µl mit Centriprep-Konzentratoren (30 kD) eingeengt und mit dem o.g. Imidazolpuffer auf 6 ml verdünnt. Es wurde hier Protein dieser Reinigungsstufe verwendet, da mit einem hohen Aktivitätsverlust durch die Chromatofokussierung zu rechnen war, und im HighQ-Eluat die höchste Aktivität (pkat/ml) vorhanden war. Die Säule wurde mit 10 ml Startpuffer nachgewaschen und mit 40 ml Polybuffer 74 (1:8 mit Millipore-Wasser verdünnt, pH 4) das Protein von der Säule eluiert. Es wurden Fraktionen von 1 ml aufgefangen, wobei zur Erhaltung der Aktivität bei niedrigen pH-Werten in jeder 2. Fraktion 100 µl 0,8 M Tris pH 8,5 vorgelegt wurde. In den dazwischenliegenden Fraktionen wurde der pH-Wert gemessen.

Um die aktive Fraktion zu ermitteln, wurde ein Aliquot jeder 2. Fraktion in folgendem Ansatz 30 min inkubiert:

| | |
|---|---|
| Ansätze | 200 µl Enzymlösung (auf pH 8,5 gepuffert) |
| | 30 µl Acetyl-Coenzym-A (5 nmol, darin 0,02 µCi[2-$^{14}$C]Acetyl-CoA enthalten) |
| | 5 µl 3 mM 10-Desacetyltaxuyunnanin C (15 nmol) |
| Inkubation | 30 min bei 35 °C |

Auswertung mittels Szintillationszähler nach Ausschütteln mit tert.-Butylmethylether.

Die Hauptaktivität eluierte im pH-Bereich von 5,7 und 5,47, sodass der isoelektrische Punkt der Acetyltransferase mit pH 5,6 bestimmt werden konnte.

### 4.4 Molekulargewichtsbestimmung

Zur Bestimmung des Molekulargewichts der Acetyltransferase wurden zwei verschiedene Methoden eingesetzt: die Gelfiltration an einer geeichten Gelfiltrationssäule und die SDS-Gelelektrophorese.

Erstere wurde an einer mit 50 mM Tris, pH 8,5, 10 mM 2-Mercaptoethanol äquilibrierten Biosilect-SEC 250-5-Säule (Biorad) in einer FPLC-Anlage (BioLogic Workstation, Biorad) durchgeführt, bei einer Flussrate von 0,2 ml/min. Zunächst wurde die Säule mit Proteinen, deren Molekulargewicht bekannt war, geeicht. Unter identischen Bedingungen wurden dann 1,5 ml HighTrapBlue-Eluat, das mit Centriprep-Konzentratoren (2 ml, Membran 10 kD) auf 100 µl (3360 pkat, 50 µg Protein) eingeengt worden war, über die Säule eluiert. Die Fraktionsgröße betrug 250 µl und die Aktivität des Eluatswurde durch Inkubation folgender Ansätze (185 µl Gesamtvolumen) ermittelt.

| | |
|---|---|
| Ansätze | 50 µl 0,8 M Tris, pH 8,5 |
| | 100 µl Eluat |
| | 30 µl Acetyl-CoA (5 nmol, darin zusätzlich 0,02 µCi [2-$^{14}$C]AcetylCoA enthalten) |
| | 5 µl 3 mM 10-Desacetyltaxuyunnanin C (15 nmol) |
| Inkubation | 30 min bei 35 °C |

Die Auswertung erfolgte mittels Szintillationszähler nach Ausschütteln mit tert.-Butylmethylether.

Für die Acetyltransferase wurde mit dieser Methode ein Molekulargewicht von 72 kD errechnet.

Zur Überprüfung dieses Wertes wurde die denaturierende SDS-Gelelektrophorese eingesetzt. Dazu wurden 0,3 µg homogenes Protein in einem 10 %-igen SDS-Gel parallel mit Markerproteinen mit bekanntem Molekulargewicht (Rainbowmarker) chromatographiert. Durch Silberfärbung wurden die Proteine sichtbar gemacht, sodass durch Vergleich der Rf-Werte der Eichproteine und der Acetyltransferase für letztere ein Molekulargewicht von 70,8 kD bestimmt werden konnte.

### 4.5 $K_M$-Wert Bestimmung

#### Bestimmung des $K_M$-Wertes für 10-Desacetytaxuyunnanin C

Um Aufschluss über die Affinität der Acetyltransferase zu 10-Desacetyltaxuyunnanin C zu erhalten, wurde der $K_M$-Wert mit dem Phenylsepharose-Eluat bestimmt.

| | |
|---|---|
| Ansätze | 50 µl 0,8 M Tris, pH 8,5<br>100 µl Acetyltransferase (0,25 pkat, 40 ng Protein, 225-fach angereichert)<br>30 µl Acetyl-Coenzym-A (5 nmol, darin zusätzlich 0,02 µCi[2-$^{14}$C]Acetyl-CoA)<br>10 µl 10-Desacetyltaxuyunnanin C<br>(Endkonzentration: 0,1/0,3/1/3/5710/30/50/100/200/300/500 µM) |
| Inkubation | 30 min bei 35 °C; Auswertung der mit tert.-Butylmethylether<br>extrahierten Radioaktivität mittels Szintillationszähler. |

Durch doppelt reziproke Auftragung der Messergebnisse nach LINEWEAVER und BURK konnte der $K_M$-Wert mit 23 µM für 10-Desacetyltaxuyunnanin C graphisch ermittelt werden.

### 4.6 Bestimmung des $K_M$-Wertes für Acetyl-Coenzym-A

Die Affinität der Acetyltransferase zu Acetyl-Coenzym-A lässt sich durch den $K_M$-Wert beschreiben, der mit dem Phenylsepharose-Eluat ermittelt wurde.

| | |
|---|---|
| Ansätze | 50 µl 0,8 M Tris, pH 8,5<br>100 µl Acetyltransferase (0,25 pkat, 40 ng Protein, 225-fach angereichert)<br>30 µl Acetyl-Coenzym-A und $H_2O$<br>(Endkonzentration2,1/2,3/3/5/12/32/52/102/152/202/302/502µM,wobei jeweils 2 µM (40 000 cpm)<br>[2-$^{14}$C]AcetylCoA enthalten waren)<br>5 µl 3 mM 10-Desacetylttaxuyunnanin C (15 nmol) |
| Inkubation | 30 min bei 35 °C, anschließend erfolgte die Auswertung durch Ausschütteln mit tert.-Butylmethylether und Szintillationszähler. Durch doppelt reziproke Auftragung der Messwerte nach LINEWEAVER und BURK konnte der $K_M$-Wert für Acetyl-Coenzym-A mit 61 µM bestimmt werden. |

### 4.7 Umsatzzahl $k_{cat}$

Die Umsatzzahl (Turnover) beschreibt die Reaktionsgeschwindigkeit einer enzymatischen Reaktion, indem sie angibt, wieviele Moleküle Substrat pro Sekunde von einem Molekül Enzym umgesetzt werden.

Zur Bestimmung des Turnover wurde Phenylsepharose-Eluatverwendet. Die darin enthaltende Konzentration an Acetyltransferase wurde durch Auftrennen des Eluat mittels SDS-Polyacrylamidgelelektrophorese und anschließender Silberfärbung bestimmt. In den benachbarten Spuren des Geles waren bekannte Mengen Rinderserumalbumin als Vergleichskonzentrationen aufgetragen worden.

Mit 300 µl Enzymlösung, entsprechend 18 ng Acetyltransferase, 10-Desacetyltaxuyunnanin C und Acetyl-Coenzym-A wurde eine Kinetik des Umsatzes aufgenommen, die im Bereich von 5 bis 30 min linear verlief.

| | |
|---|---|
| Ansätze | 50 µl 0,8 M Tris, pH 8,5<br>300 µl Acetyltransferase (18 ng Acetyltransferase, 0,76 pkat)<br>30 µl Acetyl-Coenzym-A (5 nmol, darin 0,02 µCi[2-$^{14}$C]AcCoA)<br>5 µl 3 mM 10-Desacetyltaxuyunnanin C (15 nmol) |
| Inkubation | 30 min bei 35 °C |

Die Inkubationsansätze wurden anschließend mit tert.-Butylmethylether ausgeschüttelt und mit dem Szintillationszähler ausgewertet. Aus den Messwerten konnte der Umsatz (pmol) errechnet werden. Dividiert man das Molekulargewicht (72 kD) durch die Enzymmenge (18 ng), erhält man eine Enzymkonzentration von 0,25 pmol im Ansatz.

Die Enzymaktivität berechnet man, indem man den Umsatz pro Zeiteinheit (sec) ermittelt. Der Quotient von Enzymaktivität (0,05 pmol/sec) und - konzentration (0,25 pmol) ergibt für den Turnover der Acetyltransferase einen Wert von 0,2 kat/mol homogenes Enzym (mol/sec/mol Enzym).

**Turnover = Umsatzzahl $k_{cat}$:** 0,05 pmol/sec : 0,25 pmol = **0,2 kat/mol**

Dies entspricht einem Umsatz von 0,2 Mol Substrat pro Sekunde pro Mol (72 kg) Enzym bei 35 °C, pH 8,5 und optimalen Substratmengen (60 Mol 10-DesacetyltaxuyunnaninC, 20 Mol Acetyl-CoA).

### 4.8 Kinetisches Optimum

Unter physiologischen Bedingungen ist die Substratkonzentration im Verhältnis zur Enzymkonzentration sehr gering. Nur ein kleiner Teil der aktiven Zentren des Enzyms ist besetzt, sodass die Menge der von Substrat unbesetzten Enzymen [E] annähernd der Gesamtenzymmenge [$E_0$] entspricht.

Liegt die Substratkonzentration [S] weit unter $K_M$, der Konzentration, bei der die Initialgeschwindigkeit der Reaktion halbmaximal ist, läuft die enzymatische Reaktion deutlich langsamer als durch die Umsatzzahl $k_{cat}$ ausgedrückt.

Zur Charakterisierung eines Enzyms unter diesen Bedingungen verwendet man den Quotienten $k_{cat}/K_M$. Multipliziert man diesen Wert mit der Substratkonzentration [S] und der Gesamtenzymmenge [$E_0$] erhält man die Reaktionsgeschwindigkeit.

$$v = (k_{cat}/k_M)\ [S]\ [E_0]$$

Dabei ist zu beachten, dass die Diffusionsgeschwindigkeit von im wässrigen Milieu gelösten Molekülen maximal $10^8$ bis $10^9$ betragen kann. Auch bei sehr schnellen Enzymen wird somit die Reaktionsgeschwindigkeit limitiert, da das Substrat nicht schneller zum Enzym gelangen kann.

Für die Acetyltransferase berechnet sich das kinetische Optimum wie folgt. $K_M$(10-Desacetyltaxuyunnanin C) = 23 µM, $k_{cat}$ = 0,2 kat/mol
$k_{cat}/K_m = 0{,}2\ mol\ s^{-1}\ mol^{-1}/23 \cdot 10^{-3}[M] = 8{,}7\ s^{-1}\ M^{-1}$

<u>Beispiel 5</u>

**Substratspezifität**

Zur Überprüfung der Substratspezifität der gereinigten Acetyltransferase wurden verschiedene Verbindungen mit Taxangrundgerüst als Substrat eingesetzt.

- 10-Desacatyltaxuyunnanin C
- 14-Desacetyltaxuyunnanin C
- 10,14-Desacetyltaxuyunnanin C
- 2,10,14-Desacetyltaxuyunnanin C
- 5,10,14-Desacetyltaxuyunnanin C
- 2,5,10,14-Desacetyltaxuyunnanin C
- 2,14-Desacetyltaxuyunnanin C
- 5,14-Desacetyltaxuyunnanin C
- 2,5-Desacetyl-10,14-desacetyltaxuyunnanin C
- 10-Desacetylbaccatin III (= 10-DAB III)
- 10-Desacetyltaxol
- 10-Desacetylcephalomannin
- 10-Epi-10-DAB-III
- 19-Hydroxy-10-DAB-III
- 14-Hydroxy-10-DAB-III
- 7-TES-10-DAB-III
- 7-BOC-10-DAB-III

Es stellte sich heraus, dass von den eingesetzten Substraten nur Taxane mit einer Hydroxylgruppe an Position C-10 umgesetzt werden können. Taxanderivate mit acetylierter Position C-10 aber freien Hydroxylgruppen an anderen Kohlenstoffen wurden von der gereinigten Acetyltransferase nicht als Substrat akzeptiert. Ist jedoch der Zugang zu C-10 durch raumfüllende Substituenten blockiert, wie im Falle von 10-Desacetyltaxol und 10-Desacetylcephalomannin, unterbleibt eine Acetylierung.

Bei der Berechnung der Umsatzraten, bezogen auf die Umsatzrate von 10-Desacetyltaxuyunnanin C stellte sich heraus, dass alle Taxanuyunnanin C-Derivate mit freier Hydroxylgruppe in gleichem Ausmaß umgesetzt wurden. Das 10-DAB wird mit einer Umsatzrate von 85 % im Vergleich zu 10-Desacetyltaxuyunnain C zu Baccatin III umgesetzt.

Der Umsatz verschiedener Substrate, bezogen auf den Umsatz von Desacetyltaxuyunnanin C ist in der folgenden Tabelle dargestellt.

Umsatz verschiedener Substrate, bezogen auf den Umsatz von 10-Desacetyltaxuyunnanin C

10-Decetyltaxuyunnanin C

Enzym: Phenylsepharose-Eluat (225-fach gereinigt)

| Substrat | Umsatz [%] | pkat |
|---|---|---|
| 10-Desacetyltaxuyunnanin C | 100 | 1,12 |
| 10-Desacetylbaccatin III | 80 | 0,9 |
| 10,14-Desacetyltaxuyunnanin C | 102 | 1,14 |
| 2,10,14-Desacetyltaxuyunnanin C | 81 | 0,91 |
| 5,10,14-Desacetyltaxuyunnanin C | 88 | 0,99 |
| 2,5,10,14-Desacetyltaxuyunnanin C | 53 | 0,59 |
| 10-epi-10-DAB III | 0 | 0 |
| 19-Hydroxy-10-DAB III | 38 | 0,43 |
| 14-Hydroxy-10-DAB III | 97 | 1,09 |
| 7-TES-10-DAB III | 0 | 0 |
| 7-BOC-10-DAB III | 2 | 2 |

**Patentansprüche**

**1.** Verfahren zur Herstellung von Baccatin oder/und Baccatinderivaten,
**dadurch gekennzeichnet,**
**dass** man 10-Desacetylbaccatin oder ein 10-Desacetylbaccatinderivat in Gegenwart eines isolierten Enzyms und eines Acetyldonors umsetzt, wobei als Enzym eine Acetyltransferase mit einem Molekulargewicht von 70 bis 72 kD, bestimmt durch SDS-PAGE mit einem isoelektrischen Punkt von pH 5,4 bis 5,8 und eine Michaelis Menten Konstante $K_M$ für Acetyl-Coenzym A von 55 bis 65 µM verwendet wird, die aus Zellkulturen von Taxus chinensis erhältlich ist.

**2.** Verfahren nach Anspruch 1,
**dadurch gekennzeichnet,**
**dass** man Baccatin-III aus 10-Desacetylbaccatin-III herstellt.

**3.** Verfahren nach Anspruch 1,
**dadurch gekennzeichnet,**
**dass** man 14-Hydroxybaccatin-III aus 14-Hydroxy-10-desacetylbaccatin-III herstellt.

**4.** Verfahren nach Anspruch 1,
**dadurch gekennzeichnet,**
**dass** man Taxuyunannin C aus 10-Desacetyltaxuyunnanin C herstellt.

**5.** Verfahren nach einem der vorhergehenden Ansprüche,
**dadurch gekennzeichnet,**
**dass** die Umsetzung in Gegenwart von Acetyl-Coenzym-A als Acetyldonor durchgeführt wird.

**6.** Enzym,
**dadurch gekennzeichnet,**
**dass** es

a) 10-Desacetylbaccatin-III in Gegenwart eines Acetyldonors, insbesondere Acetyl-Coenzym A selektiv an Position 10 acetyliert,
b) ein Molekulargewicht von 70 bis 72 kD aufweist, bestimmt durch SDS-PAGE und
c) einen isoelektrischen Punkt von pH 5,4 bis 5,8 aufweist,
d) eine Michaelis Menten Konstante $K_M$ für Acetyl-Coenzym A von 55 bis 65 μM aufweist,
e) eine 10-Hydroxytaxa-O-acetyltransferase ist und
f) aus Zellkulturen von Taxus chinensis erhältlich ist.

**7.** Enzym nach Anspruch 6,
**dadurch gekennzeichnet,**
**dass** es in einer Reinheit von > 50 % vorliegt.

**8.** Enzym nach Anspruch 7,
**dadurch gekennzeichnet,**
**dass** es in einer Reinheit von > 90 % vorliegt.

**9.** Verfahren zur Herstellung eines Enzyms nach einem der Ansprüche 6 bis 8,
**dadurch gekennzeichnet,**
**dass** man das Enzym aus Taxus chinensis durch Reinigung isoliert, und nach jeder Reinigung die Fraktionen bestimmt, in denen das Enzym vorliegt, indem man 10-Desacetylbaccatin oder ein 10-Desacetylbaccatinderivat und einen Acetyldonor zugibt und das gebildete Acetylierungsprodukt nachweist.

**10.** Verfahren nach Anspruch 9,
**dadurch gekennzeichnet,**
**dass** die Reinigungsverfahren die Verwendung einer HighQ-Säule umfassen.

**11.** Verfahren nach einem der Ansprüche 9 oder 10,
**dadurch gekennzeichnet,**
**dass** 10-Desacetylbaccatin-III oder 10-Desacetyltaxuyunnanin C für den Nachweis von Enzym enthaltenden Fraktionen verwendet wird.

**12.** Verfahren nach einem der Ansprüche 9 bis 11,
**dadurch gekennzeichnet,**
**dass** Acetylcoenzym A als Acetyldonor verwendet wird.

**13.** Verfahren nach einem der Ansprüche 9 bis 12,
**dadurch gekennzeichnet,**
**dass** der Nachweis des gebildeten Acetylierungsprodukts über eine radioaktive Markierung durchgeführt wird.

**14.** Verfahren nach einem der Ansprüche 9 bis 12,
**dadurch gekennzeichnet,**
**dass** der Nachweis des gebildeten Acetylierungsproduktes über eine Markierung mit einem schweren Isotop erfolgt.

**15.** Verfahren zur Herstellung von Taxol oder/und Taxolderivaten,
**dadurch gekennzeichnet,**
**dass** man zunächst Baccatin oder ein Baccatinderivat nach einem der Ansprüche 1 bis 5 herstellt und dieses durch Veresterung der OH-Gruppe in Position 13 der Baccatinderivate mit einer geeigneten Säure zu Taxol oder einem Taxoiderivat umsetzt.

## Revendications

**1.** Procédé de préparation de baccatine et/ou de dérivés de baccatine, **caractérisé en ce que** l'on fait réagir la 10-désacétylbaccatine ou un dérivé de 10-désacétylbaccatine en présence d'une enzyme isolée et d'un donneur acétyle, en utilisant comme enzyme une acétyltransférase ayant un poids moléculaire de 70 à 72 kD, déterminé par SDS-PAGE, avec un point isoélectrique de pH 5,4 à 5,8 et une constante de Michaelis-Menten $K_M$ pour la coenzyme acétyle A de 55 à 65 μM, pouvant être obtenue à partir de cultures cellulaires de Taxus chinensis.

2. Procédé selon la revendication 1, **caractérisé en ce que** l'on prépare la baccatine-III à partir de la 10-désacétylbaccatine-III.

3. Procédé selon la revendication 1, **caractérisé en ce que** l'on prépare la 14-hydroxybaccatine-III à partir de la 14-hydroxy-10-désacétylbaccatine-III.

4. Procédé selon la revendication 1, **caractérisé en ce que** l'on prépare la taxuyunannine C à partir de la 10-désacétyltaxuyunannine C.

5. Procédé selon l'une quelconque des revendications précédentes, **caractérisé en ce que** la réaction est réalisée en présence de la coenzyme acétyle A à titre de donneur acétyle.

6. Enzyme, **caractérisée en ce qu'**elle

   a) acétyle sélectivement en position 10 la 10-désacétylbaccatine-III en présence d'un donneur acétyle, en particulier la coenzyme acétyle A,
   b) présente un poids moléculaire de 70 à 72 kD, déterminé par SDS-PAGE et
   c) présente un point isoélectrique de pH 5,4 à 5,8,
   d) présente une constante de Michaelis Menten $K_M$ pour la coenzyme acétyle A de 55 à 65 µM,
   e) est une 10-hydroxytaxa-O-acétyltransférase et
   f) peut être obtenue à partir de cultures cellulaires de Taxus chinensis.

7. Enzyme selon la revendication 6, **caractérisée en ce qu'**elle présente une pureté > 50 %.

8. Enzyme selon la revendication 7, **caractérisée en ce qu'**elle présente en pureté > 90 %.

9. Procédé de préparation d'une enzyme selon l'une quelconque des revendications 6 à 8, **caractérisé en ce que** l'on isole l'enzyme provenant de Taxus chinensis par purification et que l'on détermine après chaque purification les fractions dans lesquelles l'enzyme est présente, en ajoutant la 10-désacétylbaccatine ou un dérivé de 10-désacétylbaccatine et un donneur acétyle et en détectant le produit d'acétylation ainsi formé.

10. Procédé selon la revendication 9, **caractérisé en ce que** les procédés de purification comprennent l'utilisation d'une colonne HighQ.

11. Procédé selon l'une quelconque des revendications 9 ou 10, **caractérisé en ce que** la 10-désacétylbaccatine ou la 10-désacétyltaxuyunnanine C est utilisée pour la détection des fractions contenant l'enzyme.

12. Procédé selon l'une quelconque des revendications 9 à 11, **caractérisé en ce que** la coenzyme acétyle A est utilisée comme donneur acétyle.

13. Procédé selon l'une quelconque des revendications 9 à 12, **caractérisé en ce que** la détection du produit d'acétylation formé s'effectue par le biais d'un marquage radioactif.

14. Procédé selon l'une quelconque des revendications 9 à 12, **caractérisé en ce que** la détection du produit d'acétylation formé s'effectue par un marquage avec un isotope lourd.

15. Procédé de préparation de taxol ou/et dérivés de taxol, **caractérisé en ce que** l'on prépare d'abord la baccatine ou un dérivé de baccatine selon l'une quelconque des revendications 1 à 5 et que l'on convertit celui-ci/celle-ci en taxol ou en un dérivé de taxol avec un acide approprié par estérification du groupe OH en position 13 des dérivés de baccatine.

### Claims

1. Process for preparing baccatin and/or baccatin derivatives, **characterized in that** 10-deacetylbaccatin or a 10-deacetylbaccatin derivative is reacted in the presence of an isolated enzyme and an acetyl donor, the enzyme being an acetyl transferase having a molecular weight of from 70 to 72 kD, determined by SDS-PAGE, with an isoelectric point of from pH 5.4 to 5.8 and a Michaelis Menten constant $K_M$ for acetyl coenzyme A of from 55 to 65

μM, which acetyl transferase is obtainable from Taxus chinensis cell cultures.

**2.** Process according to Claim 1, **characterized in that** baccatin III is prepared from 10-deacetylbaccatin III.

**3.** Process according to Claim 1, **characterized in that** 14-hydroxybaccatin III is prepared from 14-hydroxy-10-deacetylbaccatin III.

**4.** Process according to Claim 1, **characterized in that** taxuyunnanin C is prepared from 10-deacetyltaxuyunnanin C.

**5.** Process according to any of the preceding claims, **characterized in that** the reaction is carried out in the presence of acetyl coenzyme A as acetyl donor.

**6.** Enzyme, **characterized in that** it

a) acetylates 10-deacetylbaccatin III in the presence of an acetyl donor, in particular acetyl coenzyme A, selectively at position 10,
b) has a molecular weight of from 70 to 72 kD, determined by SDS-PAGE and
c) has an isoelectric point of from pH 5.4 to 5.8,
d) has a Michaelis Menten constant $K_M$ for acetyl coenzyme A of from 55 to 65 μM,
e) is a 10-hydroxytaxa-O-acetyl transferase and
f) is obtainable from Taxus chinensis cell cultures.

**7.** Enzyme according to Claim 6, **characterized in that** it is present in a purity of > 50%.

**8.** Enzyme according to Claim 7, **characterized in that** it is present in a purity of > 90%.

**9.** Process for preparing an enzyme according to any of Claims 6 to 8, **characterized in that** the enzyme is isolated from Taxus chinensis by purification and after each purification the fractions are determined in which the enzyme is present by adding 10-deacetylbaccatin or a 10-deacetylbaccatin derivative and an acetyl donor, and the acetylation product formed is detected.

**10.** Process according to Claim 9, **characterized in that** the purification processes include the use of a HighQ column.

**11.** Process according to any of Claims 9 or 10, **characterized in that** 10-deacetylbaccatin III or 10-deacetyltaxuyunnanin C is used for detecting the enzyme-containing fractions.

**12.** Process according to any of Claims 9 to 11, **characterized in that** the acetyl donor used is acetyl coenzyme A.

**13.** Process according to any of Claims 9 to 12, **characterized in that** the acetylation product formed is detected using radioactive labelling.

**14.** Process according to any of Claims 9 to 12, **characterized in that** the acetylation product formed is detected using labelling with a heavy isotope.

**15.** Process for preparing taxol and/or taxol derivatives, **characterized in that** initially baccatin or a baccatin derivative according to any of Claims 1 to 5 is prepared, and this is reacted by esterification of the OH group in position 13 of the baccatin derivatives with a suitable acid to give taxol or a taxol derivative.